# EUROPÄISCHE PATENTSCHRIFT

(11) **EP 2 578 262 B1**
(45) Veröffentlichungstag und Bekanntmachung des Hinweises auf die Patenterteilung: **08.03.2017**
(21) Anmeldenummer: 12186337.7
(22) Anmeldetag: 27.09.2012
(51) Int. Cl.: A61M 16/06

(54) **Sauerstoffverteiler für den Einsatz in der Sauerstoffinhalationstherapie**
Oxygen distributor for use in oxygen inhalation treatment
Distributeur d'oxygène utilisable dans le traitement par inhalation d'oxygène

(30) Priorität: 06.10.2011 DE 202011106377 U
(43) Veröffentlichungstag der Anmeldung: 10.04.2013
(73) Patentinhaber: Heinen + Löwenstein GmbH & Co. KG, 56130 Bad Ems (DE)
(72) Erfinder: Köhler, Dieter, Prof., 57392 Schmallenberg (DE); Löwenstein, Reinhard, 56130 Bad Ems (DE)
(74) Vertreter: Marx, Thomas

(56) Entgegenhaltungen:
- EP-A2- 2 140 901
- DE-C1- 4 105 672
- US-A- 2 499 650
- US-A- 4 406 283
- US-A1- 2007 163 600
- US-A1- 2011 232 649

## Beschreibung

Die vorliegende Erfindung betrifft einen Sauerstoffverteiler für den Einsatz in der Sauerstoffinhalationstherapie und insbesondere einen Sauerstoffverteiler, wie er im Oberbegriff des Patentanspruchs 1 definiert ist und der keine Rohrelemente aufweist, die im Gebrauch in die Nasenlöcher eines Patienten einzuführen sind.

Ein solcher Sauerstoffverteiler ist aus der DE 41 05 672 C1 bekannt. Derartige Systeme bieten im Vergleich zu Verteilersystemen mit den erwähnten Rohrelementen den Vorteil eines verbesserten Tragekomforts und werden von den Patienten als weniger lästig empfunden.

Problematisch hat sich bei derartigen Sauerstoffverteilern jedoch herausgestellt, dass sich in Abhängigkeit der jeweiligen Gesichtskontur des Patienten ein Abkippen des Verteilers zum Gesicht hin oder vom Gesicht weg nicht ohne Weiteres vermeiden lässt. Damit kann eine zuverlässige Sauerstoffzufuhr, sei es zur Nase und/oder zum Mund, nicht ohne Weiteres sichergestellt werden oder aber es müssen zusätzliche Befestigungen der Sauerstoff zuführenden Leitungen durch Klebestreifen am Patienten vorgenommen werden, die wiederum als unkomfortabel und lästig empfunden werden.

Vor diesem Hintergrund besteht die Aufgabe der vorliegenden Erfindung darin einen eingangs genannten Sauerstoffverteiler ohne Rohrelemente zum Einführen in die Nasenlöcher eines Patienten derart weiter zu bilden, dass ein Abkippen des Sauerstoffverteilers im Gebrauch auch ohne Verwendung weiterer Befestigungsmittel zuverlässig vermieden und daher eine verlässliche Sauerstoffinhalationstherapie gewährleistet werden kann.

Diese Aufgabe wird durch einen Sauerstoffverteiler mit den Merkmalen des Patentanspruchs 1 gelöst. Vorteilhafte Weiterbildungen der vorliegenden Erfindung finden sich in den Unteransprüchen.

Der vorliegenden Erfindung liegt dabei der Gedanke zu Grunde die Anbindung der Sauerstoffzuführung, d. h. der flexiblen Leitungen an das Anschlusselement, gelenkig zu gestalten. Vorzugsweise erfolgt dies derart, dass zumindest eine Verschwenkbarkeit der Sauerstoffzuführung, d. h. der jeweiligen flexiblen Leitung, zum Anschlusselement (in der nächsten Umgebung des Anschlusselements) um eine Achse zumindest parallel zur Mittelachse der unter der Nase zu positionierenden Austrittsöffnungen des Verwirblers möglich ist. Optimalerweise wird dies durch ein Kugelgelenk realisiert, so dass eine optimale Anpassung an die jeweilige Gesichtskontur vorgenommen werden kann.

Dementsprechend schlägt die vorliegende Erfindung einen Sauerstoffverteiler, insbesondere für den Einsatz in der Sauerstoffinhalationstherpaie vor, der einen Grundkörper mit einer Auflagefläche zum Positionieren des Sauerstoffverteilers in einem Bereich zwischen Mund und Nase eines Patienten aufweist. Mit anderen Worten liegt die Auflagefläche im Gebrauch auf der Oberlippe des Patienten auf. Ferner ist ein Verwirbler vorgesehen, der sich von einer der Auflagefläche entgegengesetzten Seite des Grundkörpers weg erstreckt und wenigstens eine Öffnung aufweist, die im Gebrauch unter der Nase positioniert und zur Nase des Patienten hin gerichtet ist sowie ein Anschlusselement zum Anschließen einer Sauerstoffzuführung, z. B. einer Leitung, an den Verwirbler aufweist. Damit schlägt die vorliegende Erfindung einen Sauerstoffverteiler vor, der keine Rohrelemente zum Einführen in die Nasenlöcher eines Patienten aufweist, sondern der Sauerstoff im Gebrauchsfall aus der Öffnung unter Nase austreten und eingeatmet werden kann. Erfindungsgemäß ist ein Gelenk zur gelenkigen Verbindung des Anschlusselements und der Sauerstoffzuführung, sprich der flexiblen Leitung vorgesehen, das eine Verschwenkung um zumindest eine Achse in der Draufsicht auf dem Sauerstoffverteiler, das heißt im Wesentlichen senkrecht zur Auflagefläche, im Wesentlichen parallel zur Mittelachse der Öffnung ermöglicht. Anders ausgedrückt, erstreckt sich die Mittelachse der Öffnung im Wesentlichen parallel zur Auflagefläche und das Gelenk ermöglicht eine Verschwenkbarkeit zumindest um eine Achse parallel zur Auflagefläche, das heißt parallel zur Oberlippe des Patienten, wodurch die Sauerstoffzuführung, ausgehend von der Oberlippe, an die Gesichtskontur angepasst, gegebenenfalls mit dieser in Kontakt verläuft, und somit auch ohne in die Nase eingeführte Rohrelemente den Sauerstoffverteiler sicher in Position hält. Dadurch kann ein Abkippen der Auflagefläche und damit des Verwirblers zuverlässig verhindert werden, insbesondere weil die Sauerstoffzuführung so vom Anschlusselement weg geführt werden kann, dass sie sich an die Gesichtskontur anlegt und somit die Auflagefläche sicher auf der Oberfläche zwischen Oberlippe und Nase hält.

Um die Anpassungsmöglichkeit weiter zu verbessern, ist es besonders bevorzugt, dass das Gelenk wenigstens zwei Freiheitsgrade, vorzugsweise drei Freiheitsgrade aufweist. Als Gelenk mit zwei Freiheitsgraden kommt eine Kreuzgelenk oder ein Drehschubgelenk in Frage. Als Gelenk mit drei Freiheitsgraden kommt ein Plattengelenk, bevorzugt jedoch ein Kugelgelenk in Frage. Das Kugelgelenk ist insbesondere aus fertigungstechnischen Gesichtspunkten zu bevorzugen, da es sich als Kunststoffspritzgussteil fertigen lässt.

Darüber hinaus ist es bevorzugt das Anschlusselement aus zwei durch das Gelenk miteinander verbundene Teile auszubilden, von denen sich ein Teil an den Verwirbler anschließt, d. h. mit dem Verwirbler verbunden, insbesondere einstückig verbunden ist, und der andere Teil gelenkig mit dem ersten Teil in Verbindung steht. Alternativ ist es jedoch auch denkbar das Gelenk in der Sauerstoffzuführung, d. h. in der Leitung vorzusehen, wobei es optimalerweise in der Nähe des Anschlusselements zu platzieren ist. Selbstverständlich ist es auch denkbar zusätzlich zu der Ausgestaltung des Anschlusselements aus zwei gelenkig miteinander verbundenen Teilen ein weiteres Gelenk in der Sauerstoffzuführung vorzusehen, z. B. einen sogenannten "Knickschlauch" zu verwenden.

Darüber hinaus ist es zur zuverlässigen Versorgung mit Sauerstoff bei guter Dosierbarkeit beim Einatmen sowohl durch die Nase als auch durch den Mund vorteilhaft eine weitere Öffnung gegenüber der Öffnung vorzusehen, die im Gebrauch über dem Mund positioniert und zum Mund des Patienten hin gerichtet ist, wie es in der DE 41 05 672 C1 erläutert ist.

Auch ist es vorteilhaft beide Nasenflügel mit Sauerstoff zu versorgen und dabei zwei Grundkörper mit jeweils einer Auflagefläche und einem Verwirbler sowie einem Anschlusselement zum Anschließen einer Sauerstoffzuführung an den Verwirbler vorzusehen, wobei die jeweiligen Öffnungen des Verneblers im Gebrauch unter einem der Nasenlöcher der Nase positioniert und zu dem jeweiligen Nasenlöcher hin gerichtet sind. Ferner ist es zur weiter verbesserten Anpassung und zur Verbesserung des Tragekomforts vorteilhaft die Verwirbler durch einen elastischen Steg miteinander zu verbinden, wohingegen die Auflageflächen im Bereich zwischen den Verwirblern durch einen Spalt getrennt sind.

Weitere Merkmale und Vorteile der vorliegenden Erfindung, die alleinstehend oder in Kombination mit einem oder mehreren der obigen Merkmale umgesetzt werden können, sind dem Fachmann aus der folgenden Beschreibung einer bevorzugten Ausführungsform ersichtlich, die unter Bezugnahme auf die begleitenden Zeichnungen erfolgt. Diese zeigen in:
Figur 1 eine perspektivische Ansicht eines Sauerstoffverteilers gemäß einer ersten Ausführungsform der vorliegenden Erfindung;
Figur 2 eine Draufsicht des Sauerstoffverteilers aus Figur 1;
Figur 3 einen Schnitt entlang der Linie A-A aus Figur 1; und
Figur 4 einen Schnitt entlang der Linie B-B aus Figur 1.

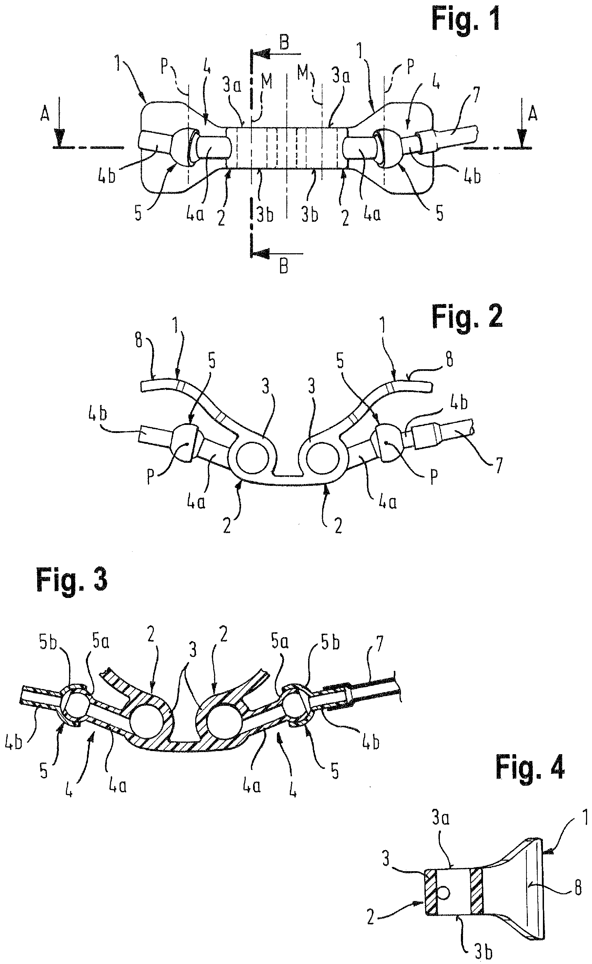

Das in Figur 1 dargestellte Ausführungsbeispiel eines erfindungsgemäßen Sauerstoffverteilers besteht aus zwei Grundkörpern 1 und zwei Verteilerelementen 2. Die Grundkörper 1 tragen jeweils das Verteilerelement 2. Die Grundkörper weisen jeweils eine Auflagefläche 8 auf, die zur Auflage und Positionierung des gesamten Sauerstoffverteilers zwischen Nase und Oberlippe eines Patienten dienen. Darüber hinaus können jeweils seitlich des Grundkörpers angeordnete Aufnahmen für ein nicht dargestelltes verstellbares Halteband vorgesehen sein, das zur zusätzlichen Befestigung des Sauerstoffverteilers festgezogen werden kann.

Die Verteilerelemente 2 bestehen jeweils aus einem Verwirbler 3 in Form eines ersten kurzen Rohrelements, dessen Längsachse im Wesentlichen parallel zur Auflagefläche verläuft. Die Verwirbler 3 sind im Wesentlichen zylindrisch gestaltet und die beiden offenen Enden bilden Öffnungen 3a und 3b. Die Öffnungen 3a und 3b der Verwirbler sind während des Gebrauchs des Sauerstoffverteilers unter der Nase und zur Nase des Patienten hin bzw. über dem Mund und zum Mund des Patienten hin angeordnet. Dabei kann der Verwirbler über seine gesamte Längen einen konstanten Querschnitt haben und somit einen zylindrischen Hohlraum in seinem inneren Umschließen. Für den Durchmesser des zylindrischen Rohrelements haben sich Werte zwischen 4 und 8 mm als besonders zweckmäßig erwiesen. Die Länge des Rohrelements beträgt vorzugsweise das 1,5- bis 2-fache des Durchmessers. Darüber hinaus ist das Rohrelement entsprechend kurz gestaltet, so dass es sich nicht in die Nasenlöcher eines Patienten erstreckt, sondern davor endet. Die Auflageflächen 8 weisen in ihrer Erstreckung parallel zur Längsachse der Verwirbler 3 bevorzugt eine gleiche oder größere Dimension auf.

Zugeführt wird der Sauerstoff über eine Schlauchleitung 7, die an einem Anschlussstutzen bzw. Anschlusselement 4 angebracht wird. Die Sauerstoffleitung kann an den Haltebändern oder am Grundkörper gesichert werden oder um die Ohren des Patienten geführt sein. Das Anschlusselement 4 ist bei den dargestellten Ausführungsformen aus zwei Teilen 4a und 4b gebildet. Der Teil 4a des Anschlusselements ist ein kurzes Rohrelement, dessen Längsachse senkrecht zur Längsachse des Verwirblers 3 verläuft und besitzt einen kleineren Durchmesser als der Verwirbler 3, vorzugsweise das 0,3- bis 0,5-fache des Durchmessers des Verwirblers (2 bis 4 mm). Dieser Teil 4a ist, wie in Figur 1 dargestellt, auf der halben Höhe des Verwirblers 3 angeordnet. Wie aus Figur 2 ersichtlich ist, liegen die Längsachsen des Verwirblers 3 und des Teils 4a nicht in einer Ebene. Vielmehr mündet, wie in Figur 4 erkennbar, ein Ende des Teils 4a in den Hohlraum des Verwirblers 3 und ist zur Längsachse des Verwirblers versetzt. Vorteilhafterweise ist eine tangentiale Einströmugn gewünscht, wie dies in der DE 41 05 672 C1 erläutert wird. Durch eine tangentiale Einströmung wird in dem Verwirbler 3 ein Zyklon gebildet, wodurch das austretende Gas stabilisiert als kleine Wolke zwischen Nase und Mund durch Nase und Mund eingeatmet werde kann. Bei einer zur Längsachse mittigen bzw. ausgerichteten Einströmung ergibt sich eine turbulente Strömung.

Der Teil 4b des Anschlusselements 4 ist ebenfalls als kurzes Rohrelement ausgestaltet. Die Teile 4a und 4b des Anschlusselements 4 sind über ein Gelenk 5 miteinander verbunden. Dabei ist bei der dargestellten Ausführungsform das dem Verwirbler abgewandte Ende des ersten Teils 4a mit einer Kugel 5a ausgebildet, während das dem Element 4a zugewandte Ende des Teils 4b mit einer korrespondierenden Kugelpfanne 5b ausgestaltet ist. Dadurch erfolgt eine gelenkige Verbindung zwischen den Teilen 4a und 4b des Anschlusselements 4 mit drei Freiheitsgraden.

Die Schlauchleitung 7 ist auf das der Pfanne abgewandte Ende des zweiten Teils 4b aufgeschoben, z. B. rein kraftschlüssig bedingt durch ihre Elastizität verbunden. Durch das Gelenk 5 ist eine Verschwenkbarkeit der zwei Teile 4a und 4b um zumindest eine Achse P parallel zur Mittelachse M der Öffnungen 3a ermöglicht. Vielmehr erfolgt durch das Kugelgelenk 5 eine Verschwenkbarkeit um eine unendliche Zahl an Achsen, so dass hier eine optimale Anpassung an die Gesichtskontur realisierbar ist und damit ein Abkippen der Öffnungen relativ zu den Nasenlöchern und/oder zum Mund um eine Achse im Wesentlichen senkrecht zu den Mittelachsen M der Öffnungen 3a und 3b vermieden werden kann. Dies wird insbesondere dadurch begünstigt, dass die Abführung der Schlauchleitung 7 so vorgenommen werden kann, dass sich die Schlauchleitung 7 an die Gesichtskontur anlegt und die Verwirbler 3 in Position hält. Dadurch kann auch ohne zusätzliche Befestigungsmaßnahmen eine Sauerstoffzufuhr auf einfachste Art und Weise sichergestellt werden. Auch kann der Sauerstoffverteiler nach wie vor aus Kunststoff in einem Spritzgießverfahren und damit sehr kostengünstig hergestellt werden.

Darüber hinaus sind die Verwirbler nur über einen Steg, der elastisch ist, miteinander verbunden, so dass hier auch eine gewisse Elastizität des Verteilers, d. h. der Verwirbler zueinander um eine Achse parallel zu den Mittelachsen M gewährleistet ist, was zu einer weiteren verbesserten Anpassung führt.

Im Übrigen wird der Fachmann bezüglich der Funktionsweise des Sauerstoffverteilers insbesondere auf die DE 41 05 672 C1 verwiesen, auf deren Inhalt voll umfänglich Bezug genommen wird.

Es versteht sich jedoch, dass die vorliegende Erfindung nicht auf die beschriebene Ausführungsform beschränkt ist, sondern vielmehr im Umfang der folgenden Patentansprüche verschiedenartige Modifikationen vorgenommen werden können. So ist die vorliegende Erfindung insbesondere nicht auf die Verwendung eines Kugelgelenks beschränkt. Vielmehr können auch einfache Gelenke mit nur einem Freiheitsgrad oder Gelenke mit zwei Freiheitsgraden zum Einsatz kommen. Rein beispielhaft sein hier einfache Drehgelenke (Scharniere), Schraubgelenke, Drehschubgelenke, Plattengelenke, Kreuzgelenke und Schubgelenke genannt. Dennoch ist die Verwendung eines Kugelgelenks aufgrund der Vielzahl von Einstellungsmöglichkeiten und der einfachen Herstellbarkeit im Rahmen eines Spritzgießverfahrens bevorzugt.

## Patentansprüche

1. Sauerstoffverteiler ohne Rohrelemente zum Einführen in die Nasenlöcher eines Patienten, insbesondere für den Einsatz in der Sauerstoffinhalationstherapie, mit:
einem Grundkörper (1), der eine Auflagefläche (8) zum Positionieren des Sauerstoffverteilers in einem Bereich zwischen Mund und Nase eines Patienten aufweist,
einem Verwirbler (3), der sich von einer, der Auflagefläche entgegengesetzten Seite des Grundkörpers (1) erstreckt und wenigstens eine Öffnung (3a) aufweist, die im Gebrauch unter der Nase positioniert und zur Nase des Patienten hin gerichtet ist, und
einem Anschlusselement (4) zum Anschließen einer Sauerstoffzuführung (7) an den Verwirbler (3), **gekennzeichnet durch**
ein Gelenk (5) zur gelenkigen Verbindung des Anschlusselements (4) und der Sauerstoffzuführung (7), wobei das Gelenk (4) eine Verschwenkung um zumindest eine Achse in der Draufsicht auf den Sauerstoffverteiler, das heißt im Wesentlichen senkrecht zur Auflagefläche, im Wesentlichen parallel zur Mittelachse der Öffnung (3a) ermöglicht, so dass die Sauerstoffzuführung an die Gesichtskontur anlegbar ist und ein Abkippen der Auflagefläche und damit des Verwirblers verhinderbar ist.

2. Sauerstoffverteiler nach Anspruch 1, wobei das Gelenk (5) wenigstens zwei, vorzugsweise drei Freiheitsgrade aufweist.

3. Sauerstoffverteiler nach Anspruch 1 oder 2, wobei das Gelenk (5) ein Kugelgelenk ist.

4. Sauerstoffverteiler nach einem der vorstehenden Ansprüche, wobei das Anschlusselement (4) aus zwei durch das Gelenk miteinander verbundenen Teilen (4a,4b) gebildet ist.

5. Sauerstoffverteiler nach einem der vorstehenden Ansprüche, wobei der Verwirbler (3) gegenüber der Öffnung (3a) eine weitere Öffnung (3b) aufweist, die im Gebrauch über dem Mund positioniert und zum Mund des Patienten hin gerichtet ist.

6. Sauerstoffverteiler nach einem der vorstehenden Ansprüche, wobei zwei Grundkörper mit jeweils einer Auflagefläche (8) und einem Verwirbler (3) sowie einem Anschlusselement (4) zum Anschließen einer Sauerstoffzuführung (7) an den Verwirbler (3) vorgesehen sind, die über einen die Verwirbler (3) verbindenden, vorzugsweise elastischen Steg (9) verbunden sind.

## Claims

1. An oxygen distributor without tube elements for introducing into a patient's nostrils, particularly for use in oxygen inhalation therapy, with:
a base unit (1), which has a mounting surface (8) for positioning the oxygen distributor in an area between a patient's mouth and nose,
a swirler (3), which extends from the side of the base unit (1) opposite to the mounting surface and has at least one opening (3a), which, when in use, is positioned under the nose and directed towards the patient's nose, and
a connecting element (4) for sealing off an oxygen supply (7) to the swirler (3), **characterized by**
a joint (5) for articulated connection of the connecting element (4) and the oxygen supply (7), wherein the joint (4) permits pivoting along at least one axis in the plan view of the oxygen distributor, that is, essentially perpendicularly to the mounting surface, essentially parallel to the center line of the opening (3a), so that the oxygen supply can be placed against the contours of the face and tilting of the mounting surface, and thus the vortex flow device, can be prevented.

2. The oxygen distributor according to Claim 1, wherein the joint (5) has at least two, preferably three degrees of freedom.

3. The oxygen distributor according to Claim 1 or 2, wherein the joint (5) is a ball joint.

4. The oxygen distributor according to any of the previous claims, wherein the connecting element (4) is formed of two parts (4a, 4b) connected by the joint.

5. The oxygen distributor according to any of the previous claims, wherein the swirler (3) has a further opening (3b) opposite the opening (3a), which, when in use, is positioned over the mouth and is directed towards the patient's mouth.

6. The oxygen distributor according to any of the previous claims, wherein two base units are provided, each with a mounting surface (8) and a swirler (3), as well as a connecting element (4) for connecting an oxygen supply (7) to the swirler (3), which are connected by a preferable elastic bridge (9) connecting the vortex flow devices (3).

## Revendications

1. Distributeur d'oxygène sans éléments de tuyaux pour insertion dans les narines d'un patient, en particulier pour utilisation en oxygénothérapie, avec :
un corps de base (1) présentant une surface d'application (8) destinée à positionner le distributeur d'oxygène sur une zone s'étendant de la bouche au nez d'un patient,
un tourbillonneur (3) s'étirant d'un côté opposé de la zone de positionnement du corps de base (1) et présentant au moins une ouverture (3a) qui, lorsque le dispositif est utilisé, est positionné en direction du nez du patient, et
un élément de raccordement (4) pour l'obturation d'une alimentation en oxygène (7) sur le tourbillonneur (3), **caractérisé par**
une articulation (5) destinée à la liaison articulée de l'élément de raccordement (4) et de l'alimentation en oxygène (7), l'articulation (4) permettant le pivotement autour d'au moins un axe, le distributeur d'oxygène étant vu par le haut, c.-à-d. essentiellement verticalement à la zone de positionnement et essentiellement parallèlement à l'axe central de l'ouverture (3a), de sorte que l'alimentation en oxygène puisse être positionnée sur le contour du visage et qu'il soit possible d'empêcher le basculement de la zone de positionnement et donc du tourbillonneur.

2. Distributeur d'oxygène selon la revendication 1, l'articulation (5) présentant au moins deux, de préférence trois degrés de liberté de mouvement.

3. Distributeur d'oxygène selon la revendication 1 ou 2, l'articulation (5) étant une articulation à boule.

4. Distributeur d'oxygène selon l'une des revendications précédentes, l'élément de raccordement (4) étant composé de deux éléments (4a, 4b) reliés par l'articulation.

5. Distributeur d'oxygène selon l'une des revendications précédentes, le tourbillonneur (3) situé en regard de l'ouverture (3a) présentant une ouverture supplémentaire (3b) qui, lorsque le dispositif est utilisé, est positionnée sur la bouche et orientée vers la bouche du patient.

6. Distributeur d'oxygène selon l'une des revendications précédentes **caractérisé en ce que** deux corps de base présentant chacun une zone de positionnement (8) et un tourbillonneur (3) ainsi qu'un élément de raccordement (4) destiné au raccordement d'une alimentation en oxygène (7) au tourbillonneur (3), sont reliés par un pontet de liaison (9) de préférence élastique reliant les tourbillonneurs (3).
